(19) Europäisches Patentamt
European Patent Office
Office européen des brevets

(11) Veröffentlichungsnummer : **0 091 044**
**B1**

(12) # EUROPÄISCHE PATENTSCHRIFT

(45) Veröffentlichungstag der Patentschrift : **18.06.86**

(51) Int. Cl.⁴ : **C 07 D417/12**

(21) Anmeldenummer : **83103027.5**

(22) Anmeldetag : **26.03.83**

(54) **Verfahren zur Herstellung von 4-Hydroxy-3-(heterocyclocarbamoyl)-2H-1,2-benzothiazin-1,1-dioxiden.**

(30) Priorität : **03.04.82 DE 3212485**

(43) Veröffentlichungstag der Anmeldung : **12.10.83 Patentblatt 83/41**

(45) Bekanntmachung des Hinweises auf die Patenterteilung : **18.06.86 Patentblatt 86/25**

(84) Benannte Vertragsstaaten : **AT BE CH DE FR GB IT LI LU NL SE**

(56) Entgegenhaltungen :
**US-A- 4 074 048**
**US-A- 4 294 961**

(73) Patentinhaber : **GÖDECKE AKTIENGESELLSCHAFT**
**Salzufer 16**
**D-1000 Berlin 10 (DE)**

(72) Erfinder : **Herrmann, Wolfgang, Dr.**
**Zum Baumgarten 13**
**D-7802 Merzhausen (DE)**
Erfinder : **Geibel, Wolfram, Dr.**
**Schwarzwaldstrasse 8**
**D-7801 Reute (DE)**
Erfinder : **Satzinger, Gerhard, Dr.**
**Im Mattenbühl 7**
**D-7809 Denzlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 091 044 B1

**Beschreibung**

Aus der DE-PS 2 208 351 ist ein Verfahren zur Herstellung von 4-Hydroxy-3-(isoxazolocarbamoyl)-2H-1.2-Benzothiazin-1,1-dioxiden der allgemeinen Formel :

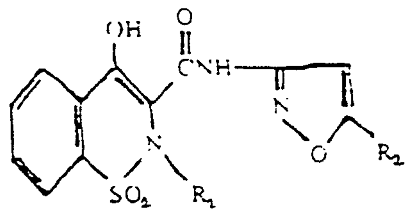

worin $R_1$ ein Wasserstoffatom oder eine Methylgruppe und $R_2$ ein Wasserstoffatom oder eine Methylgruppe bedeuten, oder deren entsprechenden Alkalimetall-, Erdalkalimetall- und Aminsalze, bekannt. Die nach dem bekannten Verfahren hergestellten Verbindungen besitzen antirheumatische Eigenschaften.

Das bekannte Herstellungsverfahren läuft beispielsweise im Falle einer Verbindung der angegebenen Formel mit $R_1$ und $R_2$ gleich einer Methylgruppe in vier getrennt ablaufenden Reaktionsstufen mit einer Gesamtausbeute von nur 40-45 % ab :

Stufe 1

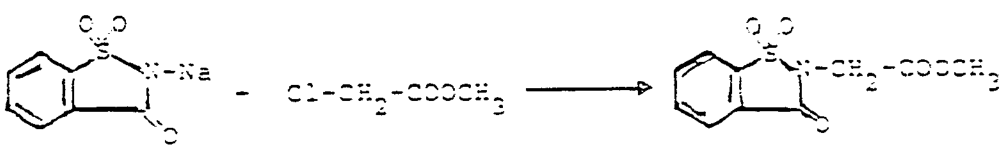

(I)

Stufe 2

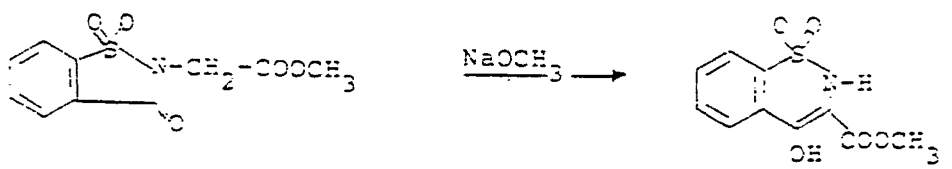


(II)

Stufe 3

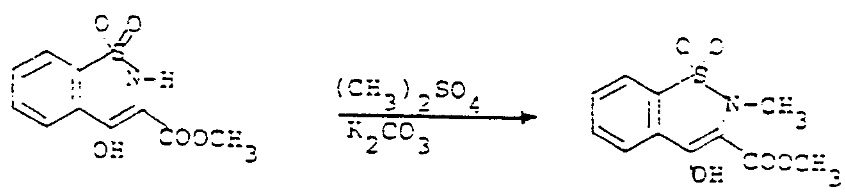


(III)

Stufe 4

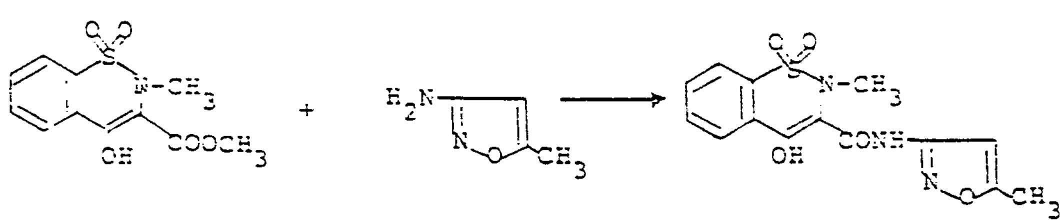

4-Hydroxy-3-(5-methyl-3-isoxazolyl-carbamoyl)-2-methyl-2H-1.2-benzothiazin-1.1-dioxid

Bei einem anderen, aus der US-PS-4 041 042 bekannten Verfahren erfolgt die Herstellung der betreffenden Verbindungen gemäß dem folgenden Reaktionsschema in fünf Stufen, wobei die jeweiligen Zwischenprodukte isoliert und in der nächsten Stufe weiterverarbeitet werden.

Stufe I

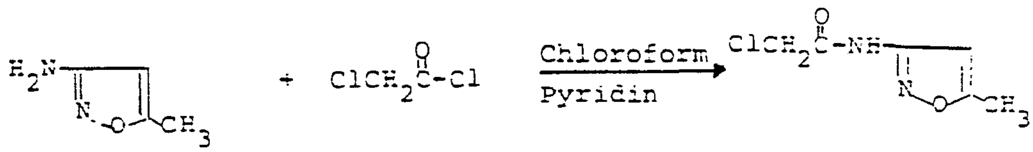


(I)

Stufe II

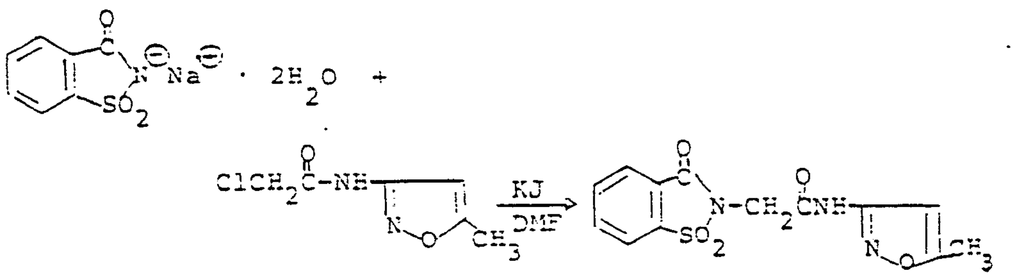


(II)

Stufe III

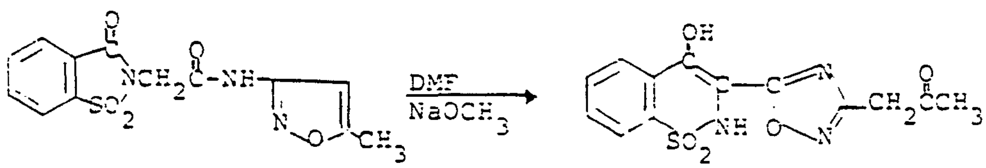


(III)

Stufe IV

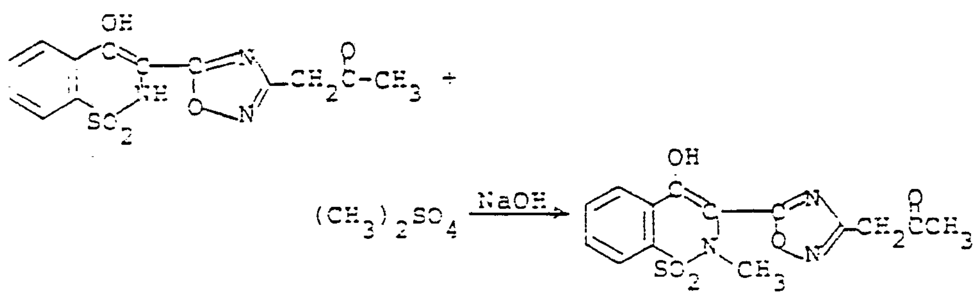


(IV)

Stufe V

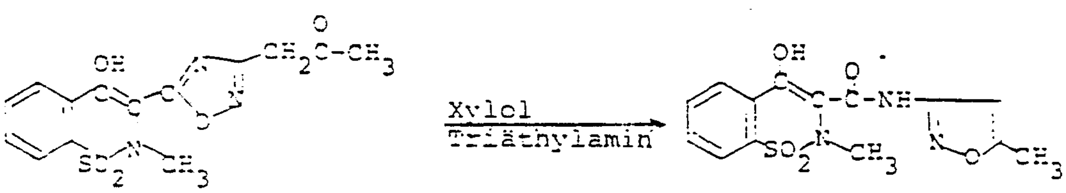


(V)

Nachteilig an dem aus der US-PS 4 041 042 bekannten Verfahren ist, daß

1. es über fünf Stufen unter Isolierung der Zwischenprodukte abläuft, weswegen ein relativ großer Arbeits-, Energie- und Zeitaufwand erforderlich ist ;
2. die Gesamtausbeute nur 27 % beträgt und
3. das Zwischenprodukt I, d. h. das Umsetzungsprodukt von 3-Amino-5-methylisoxazol und Chloracetylchlorid stark hautreizend ist.

Der Erfindung lag nun die Aufgabe zugrunde, ein Verfahren zur Herstellung von 4-Hydroxy-3-(heterocyclocarbamoyl)-2H-1,2-benzothiazin-1,1-dioxiden zu entwickeln, das bei geringerem Arbeits-, Energie- und Zeitaufwand zu höheren Ausbeuten führt, in großtechnischem Maßstab durchführbar ist und arbeitsplatzhygienischen Gesichtspunkten voll Rechnung trägt.

Der Gegenstand der Erfindung ist somit ein Verfahren zur Herstellung von 4-Hydroxy-3-(heterocyclocarbamoyl)-2H-1,2-benzothiazin-1,1-dioxiden der allgemeinen Formel

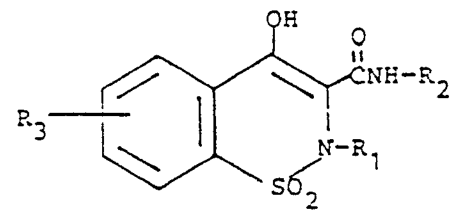

worin bedeuten :

$R_1$ ein Wasserstoffatom oder eine Methylgruppe und

$R_2$ einen gegebenenfalls durch niedere Alkyl- oder Alkoxygruppen substituierten Isoxazol-, Thiazol- oder Pyridinring

$R_3$ ein Wasserstoff- oder Halogenatom oder eine Methylgruppe

oder deren Alkalimetall-, Erdalkalimetall- oder Aminsalzen,

durch Umsetzung einer Verbindung $R_2$—$NH_2$ mit einem Halogenacetylhalogenid, Umsetzung des erhaltenen Zwischenprodukts mit Na-Benzoesäuresulfimid, Ringerweiterung des Benzoisothiazolrings zum Benzothiazinring und für den Fall, daß $R_2$ ein Isoxazolrest sein soll, Rückumlagerung des bei der Ringerweiterung gebildeten Oxadiazolringes zum Isoxazolring, dadurch gekennzeichnet, daß man (in einer Eintopfreaktion A) die Umsetzung der Verbindung $R_2$—$NH_2$ mit einem Halogenacetylhalogenid ohne Basenzusatz in siedendem Äthylacetat durchführt, das gebildete Zwischenprodukt ohne Isolierung mit Na-Benzoesäuresulfimid umsetzt und das Reaktionsprodukt isoliert, daß man (in einer Eintopfreaktion B) das Reaktionsprodukt aus der Eintopfreaktion A in einem dipolar aprotonischen Lösungsmittel unter Schutzgas mit einem stark basischen verzweigten Alkalimetall-alkoholat und danach mit soviel Säure, daß in der Reaktionslösung noch etwa 2 Äquivalente Base unneutralisiert bleiben, reagieren läßt, und daß man gegebenenfalls den Benzothiazinring in an sich bekannter Weise N-methyliert.

Die heterocyclischen Reste $R_2$ können, insbesondere durch Methyl- oder Methoxygruppen substituiert sein.

Das erfindungsgemäße Verfahren ermöglicht eine sehr einfache und kostengünstige Herstellung von Verbindungen der angegebenen Formel. Es besteht aus zwei aufeinanderfolgenden Eintopfreaktionen (im weiteren als Eintopfreaktionen A und B bezeichnet), in denen jeweils zwei bzw. drei Reaktionsschritte ohne Isolierung der Zwischenprodukte nacheinander ablaufen. Die Ausbeute beträgt in der Stufe A 94 % und in der Stufe B 82-86 % ; das entspricht einer Gesamtausbeute von 77-80 % gegenüber 40-45 % bei dem aus der DE-PS 2 208 351 bekannten Verfahren und 27 % bei dem aus der US-PS 4 041 042 bekannten Verfahren.

Das erfindungsgemäße Verfahren wird nachstehend an Hand der Herstellung von 4-Hydroxy-3-(5-methyl-3-isoxazolyl-carbamoyl)-2-methyl-2H-1,2-benzothiazin-1,1-dioxid (Isoxicam) beschrieben :

Eintopfreaktion A

Reaktionsschritt 1

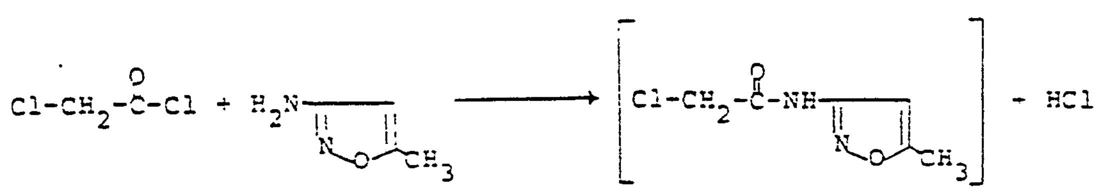

(1)

Reaktionsschritt 2

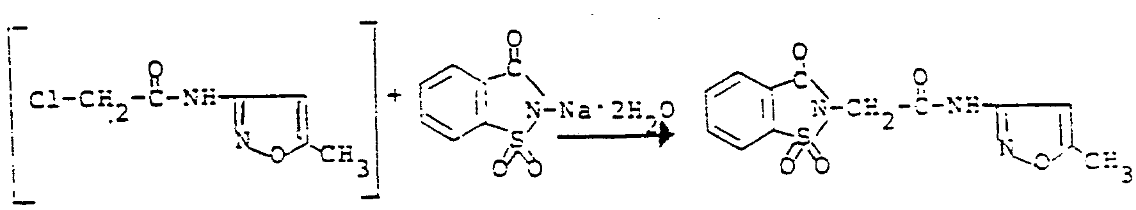

(1) (2)

In einem ersten Reaktionsschritt wird 3-Amino-5-methyl-isoxazol mit Chloracetylchlorid umgesetzt. Diese Umsetzung wurde bei dem aus der US-PS 4 041 042 bekannten Verfahren in Chloroform durchgeführt unter Zugabe von Pyridin zum Binden des entstehenden Chlorwasserstoffs. Dabei fiel

Pyridin-Hydrochlorid zusammen mit dem Umsetzungsprodukt aus der Lösung aus und mußte durch mehrfaches Waschen und Aufschlämmen in Wasser entfernt werden.

Es wurde nun überraschenderweise gefunden, daß diese Umsetzung in siedendem Äthylacetat ohne Basenzusatz durchgeführt werden kann, wobei der gebildete Chlorwasserstoff zusammen mit etwas Äthylacetat abdestilliert und so aus dem Reaktionsgemisch entfernt werden kann. Eigentlich wäre anzunehmen gewasen, daß entstandener Chlorwasserstoff von noch nicht umgesetztem Amino-isoxazol salzartig gebunden würde und dieses damit nicht zur Reaktion mit dem Säurechlorid kommen könne, was zu einer schlechten Ausbeute geführt hätte oder zu der Notwendigkeit, das Amino-isoxazol im Überschuß anzuwenden. Durch die Tatsache, daß keine Base zum Binden des Chlorwasserstoffes zugesetzt zu werden braucht und dieser praktisch vollständig abdestilliert, wird ein salzfreies Reaktionsgemisch erhalten, was wiederum nach Abdestillieren des restlichen Äthylacetats die sofortige, weitere Umsetzung mit Na-Benzoesäuresulfimid zum Produkt (2) ermöglicht. Damit ist es möglich geworden, auf die Isolierung des hautschädigenden Zwischenproduktes (1) zu verzichten. Seine völlige Umsetzung wird durch die Anwendung eines Überschusses von Na-Benzoesäuresulfimid sichergestellt. In (2) ist (1) nicht mehr nachweisbar, so daß eine Hautschädigung beim Verarbeiten von Produkt (2) nicht anzunehmen ist.

Das Endprodukt der Eintopfreaktion A wird dann durch Ausfällung mit Wasser mit einer Ausbeute von 90-95 % und in einer Reinheit von 98-99 % erhalten.

Nach dem erfindungsgemäßen Verfahren wird nun das getrocknete Reaktionsprodukt aus der Eintopfreaktion A in einer Eintopfreaktion B in Dimethylformamid als Lösungsmittel in 4-Hydroxy-3-(5-methyl-3-isoxazolylcarbamoyl)-2-methyl-2H-1,2-benzothiazin-1,1-dioxid umgewandelt.

Eintopfreaktion B

Reaktionsschritt 3

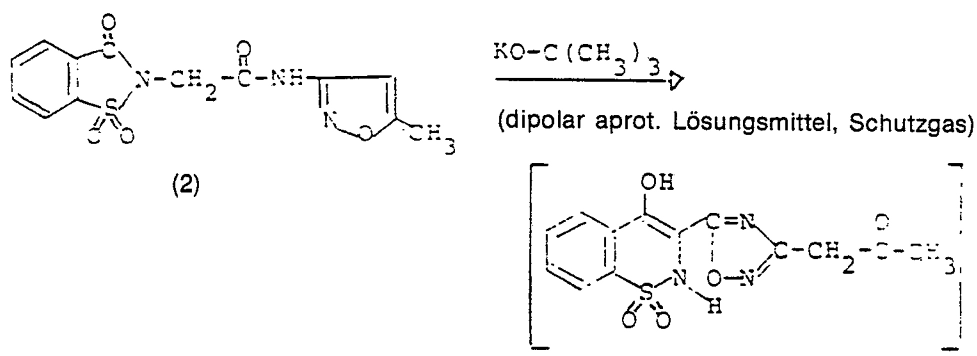


(2)

(3) (nicht isoliert)

Reaktionsschritt 4

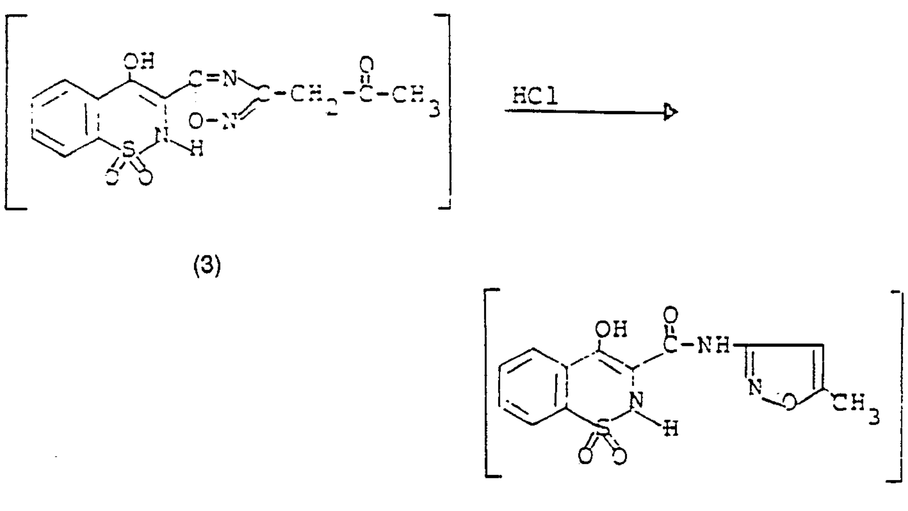


(3)

(4) (nicht isoliert)

Reaktionsschritt 5

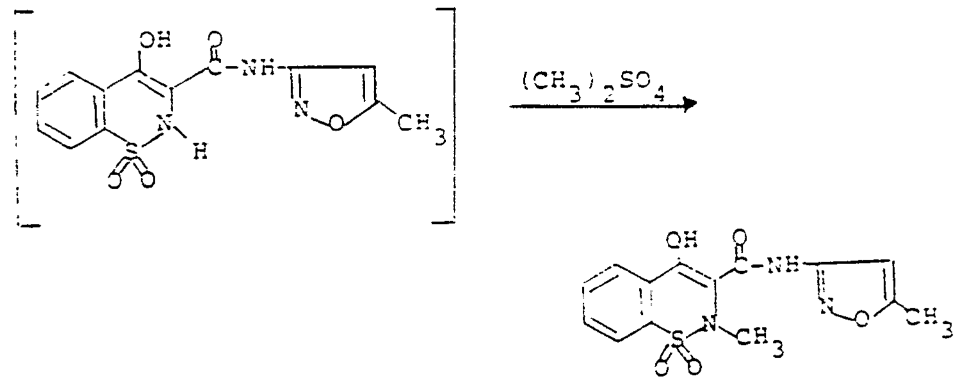


4-Hydroxy-3-(5-methyl-3-isoxazolyl-carbamoyl)-2-methyl-2H-1,2-benzothiazin-1,1-dioxid (5)

Nach dem aus der DE-PS 2 208 351 bekannten Verfahren wird die Umlagerung des Benzoisothiazolrings zum Benzothiazinring durch die Einwirkung von Natriummethylat erreicht, wobei die Ausbeute 60 % beträgt. Es wurde nun gefunden, daß die Ringerweiterung bei der Verwendung von stärkeren Basen, das sind im Sinne der vorliegenden Erfindung verzweigte Alkalimetallalkoholate, die Natriummethylat an Basizität übertreffen, wie Kalium tert. butylat, nahezu quantitativ abläuft. Wie bei der Einwirkung von Natriummethylat wird gleichzeitig zur Ringerweiterung der Isoxazolring der Seitenkette umgelagert in einen 1,2,4-Oxadiazolring (3). Diese Umlagerung muß wieder rückgängig gemacht werden, wenn man 4-Hydroxy-3-(5-methyl-3-isoxazolyl-carbamoyl)-2-methyl-2H-1,2-benzothiazin-1,1-dioxid erhalten will. Nach dem aus der US-PS 4 041 042 bekannten Verfahren geschieht das in der letzten Reaktionsstufe durch Erhitzen mit Triäthylamin in Xylol, nachdem in der vorletzten Stufe der Stickstoff des Benzothiazins methyliert worden ist. Es hat sich nun gezeigt, daß diese Rückumlagerung zu (4) im Rahmen des erfindungsgemäßen Verfahrens überraschenderweise direkt in der Reaktionslösung des Produktes (3) erreicht werden kann, wenn man eine bestimmte Menge Säure zugibt. Diese Säure kann eine anorganische oder eine organische Säure sein. Vorzugsweise wird Chlorwasserstoff benutzt, uns zwar entweder als Lösung in einem bipolar aprotonischen Lösungsmittel, bevorzugt in Dimethylformamid oder Dimethylsulfoxid oder als konzentrierte wäßrige Salzsäure verdünnt mit dem angewandten bipolar aprotonischen Lösungsmittel. Die Menge an Säure sollte so bemessen sein, daß in der Reaktionslösung noch ca. zwei Äquivalente Basen unneutralisiert vorhanden sind ; d. h. zum Beispiel bei der Verwendung von vier Äquivalenten tert.-Butylat werden zwei Äquivalente Säuren zugegeben, bei der Verwendung von drei Äquivalenten tert.-Butylat nur eines. Die Reaktionstemperatur der rasch verlaufenden Rückumlagerung (3)→(4) sollte zwischen — 5 ° und 25 °C, vorzugsweise bei + 5 °C liegen. Die Umsetzung erfolgt zweckmäßig in einer Schutzgasatmosphäre, z. B. unter Stickstoff, Argon oder Helium.

Anschließend kann in der gleichen Lösung nach Zugabe von einem Äquivalent Kaliumhydroxid die N-Methylierung von (4) zur Verbindung (5) mit Dimethylsulfat durchgeführt werden. Das Endprodukt wird durch Ansäuern und Wasserzugabe ausgefällt. Im Pilotmaßstab ist diese Eintopfreaktion (3)→(4)→(5) ohne Schwierigkeit in einem halben Tag durchzuführen.

Beispiel 1

Herstellung von 4-Hydroxy-3-(5-methyl-3-isoxazolyl-carbamoyl)-2-methyl-2H-1,2-benzothiazin-1,1-dioxid

Eintopfreaktion A

Reaktionsschritte 1 und 2

In einem 100 l Pfaudler Kessel werden
27,75 l Essigsäureäthylester vorgelegt und
1 850 g (18,85 Mole) 3-Amino-5-methyl-isoxazol eingetragen.

Unter mäßigem Rühren wird die Mischung zum Sieden gebracht, wobei Lösung eintritt. Es werden ca. 2 l Essigsäureäthylester abdestilliert, um eventuelle Wasserspuren aus Lösungsmittel und Kessel zu entfernen. Danach wird eine Mischung von
1,5 l (18,85 Mol) Chloracetylchlorid in
4,5 l Essigsäureäthylester in etwa 1 h zulaufen gelassen. Dabei wird durch leichtes Heizen die Mischung am Sieden gehalten ; der Siedepunkt fällt dabei von anfänglich 77 °C auf 72 °C. Während des Zulaufens werden ca. 11 l Essigsäureäthylester abdestilliert. Die bei der Reaktion entstehende Salzsäure entweicht aus dem Reaktionsgemisch und löst sich in dem kondensierten Essigsaureäthylester. Nach dem Zulaufen wird noch 1 h gekocht, um die Salzsäure vollständig aus der Reaktionmischung

auszutreiben. Dabei werden weitere 12 l Essigsäureäthylester abdestilliert. Bei abgestellter Heizung wird dann eine Lösung von

5 451 g (22,6 Mol) Na-Benzoesäuresulfimid × $2H_2O$ und

312 g (1,885 Mol) Kaliumjodid in

18,85 l Dimethylformamid in ca. 5 min zulaufen gelassen. Anschließend wird auf 90 °C geheizt und 1 h 45 min bei dieser Temperatur gerührt. Nach Abstellen der Heizung werden

52 l Wasser rasch zulaufen gelassen. Das nach vorübergehender klarer Lösung auskristallisierende Produkt wird abgeschleudert, mit

20 l Wasser auf der Zentrifuge gewaschen und bei 50 °C bis zur Gewichtskonstanz getrocknet.

Ausbeute : 5,7 kg = 94 % d. Theorie

Fp. : 217,6 °C $H_2O$ n. KF : < 0,1 %

HPLC : Gehalt 98-99 % Produkt (1) nicht nachweisbar (< 0,1 %)

Eintopfreaktion B

Reaktionsschritte 3, 4 und 5

Im 100 l-Pfaudler-Kessel wurden unter Stickstoff

13,5 l Dimethylformamid vorgelegt und unter leichtem Rühren

4 490 g (40 Mol) Kalium-tert-butylat eingetragen. Unter gutem Rühren und Kühlen mit Kaltwasser wurde eine unter $N_2$ hergestellte Lösung von

3 213 g (10 Mol) Reaktionsprodukt aus der Eintopfreaktion A in

11,2 l Dimethylformamid in 10 min bei ca. 25 °C zulaufen gelassen. Mit Dampf wurde auf 50 °C geheizt und 15 min gerührt. Danach wurde auf 0 °C gekühlt und eine Lösung von

729 g (20 Mol) Salzsäure gasförmig in

3,2 l Dimethylformamid unter Kühlung mit Sole in ca. 15 min bei + 5 °C zulaufen gelassen. Nach Zugabe einer Lösung von

660 g (10 Mol) Kaliumhydroxyd (85 %ig) in

1,32 l Wasser wurden unter gutem Rühren in ca. 10 min

2 522 g (20 Mol) Dimethylsulfat zulaufen gelassen. Die Temperatur stieg dabei bis auf 30 °C an. Danach wurde 30 min bei 30 °C gerührt. Zur Vernichtung von überschüssigem Dimethylsulfat wurden

1 000 ml (ca. 13,3 Mol) konzentrierte Ammoniaklösung zugegeben und 30 min bei 30 °C gerührt. Nach Zugabe von

50 l Wasser wurde eine Mischung von

3 l (37,5 Mol) konzentrierter Salzsäure in

20 l Wasser zulaufen gelassen. Es wurde auf 20 °C abgekühlt. Das Produkt wurde abgeschleudert und mit

15 l Wasser gewaschen. Bei 60 °C wurde getrocknet.

Ausbeute : 2,78 kg = 83 % d. Th. 4-Hydroxy-3-(5-methyl-3-isoxazolyl-carbamoyl)-2-methyl-2H-1,2-benzothiazin-1,1-dioxid roh.

Reinheit nach HPLC : 96 %

Fp. : 253 °C (Zersetzung)

(Beispiel 1A)

Variante II

16 g (0,05 Mol) des getrockneten Reaktionsproduktes (2) der Eintopfreaktion A werden in 56,2 ml Dimethylsulfoxid gelöst und bei Raumtemperatur unter Stickstoff und unter Rühren zu einer Lösung von 18,5 g (0,165 Mol) Kalium-tert.-butylat in 67,2 ml Dimethylsulfoxid innerhalb von 5 Minuten zugetropft. Man rührt 10 Minuten bei 55 °C, kühlt auf 5 °C ab und tropft bei gleicher Temperatur 8 ml (0,1 Mol) konzentrierte Salzsäure, die mit 8 ml Dimethylsulfoxid verdünnt wurde, zu. Das so erhaltene Reaktionsgemisch wird nach Zugabe weiterer 6,8 g (0,085 Mol) 50 %iger Natronlauge mit 10 ml (0,1 Mol) Dimethylsulfat umgesetzt, 15 Minuten nachgerührt und nach Abbruch der Reaktion mit 100 ml verdünnter Ammoniaklösung in eine Vorgabe von 500 ml verdünnter Salzsäure eingerührt.

Man erhält 10,7 g 4-Hydroxy-3-(5-methyl-3-isoxazolyl-carbamoyl)-2-methyl-2H-1,2-benzothiazin-1,1-dioxid (Reinheit nach HPLC : 95 %).

Beispiel 2

Herstellung von 4-Hydroxy-3-(2-pyridyl-carbamoyl)-2-methyl-2H-1,2-benzothiazin-1,1-dioxid

Eintopfreaktion A

Zu einer Lösung von 28,2 g (0,3 Mol) 2-Amino-pyridin in 750 ml Essigsäureäthylester werden in der

Siedehitze 23,8 ml (0,3 Mol) Chloracetylchlorid in 60 ml Essigsäureäthylester während 15 Minuten zugetropft und 15 Minuten nachreagieren gelassen. Während des Zutropfens und der Nachreaktion werden insgesamt 700 ml Essigsäureäthylester abdestilliert. Anschließend fügt man bei ca. 65 °C 144 g (0,6 Mol) Natrium-Benzoesäuresulfimid × $2H_2O$ und 5 g (0,03 Mol) Kaliumjodid in 434 ml Dimethylformamid gelöst zu und rührt 3,5 Stunden nach. Danach wird der Ansatz auf 3 l Eiswasser gegossen und mit verdünnter Ammoniaklösung neutralisiert. Beim Stehen kristallisieren 32,2 g aus (Schmelzpunkt 178 °C), die wie in Beispiel 1 isoliert werden.

Eintopfreaktion B

Bei Raumtemperatur werden unter $N_2$ zu einer gerührten Lösung von 8,97 g (0,08 Mol) Kalium-tert.-butylat in 27 ml Dimethylformamid, 6,34 g (0,02 Mol) getrocknetes Reaktionsprodukt der Eintopfreaktion A, in 19 ml Dimethylformamid gelöst, zugegeben. Das Reaktionsgemisch wird 60 Minuten bei 55 °C und 30 Minuten bei 80 °C gerührt, anschließend abgekühlt und bei Raumtemperatur mit 6 ml (0,04 Mol) halbkonzentrierter Salzsäure teilweise neutralisiert und 15 Minuten mit 5 ml (0,05 Mol) Dimethylsulfat bei 25 °C umgesetzt. Die Umsetzung wird durch Zugabe von 40 ml (0,04 Mol) 1 N Natronlauge — 15 Minuten Rühren — vervollständigt. Das Reaktionsgemisch wird schließlich mit 40 ml verdünnter Ammoniaklösung 15 Minuten gerührt, in 400 ml Wasser eingerührt und mit Salzsäure angesäuert. Man erhält 2,5 g 4-Hydroxy-3-(2-pyridyl-carbamoyl)-2-methyl-2H-1,2-benzothiazin-1,1-dioxid. Zur Reinigung wird das Produkt aus Methanol oder einem Gemisch Dichlormethan — Methanol (7 : 3) umkristallisiert. (Schmelzpunkt 199,5 °C)

## Beispiel 3

Herstellung von 4-Hydroxy-3-(2-thiazolyl-carbamoyl)-2-methyl-2H-1,2-benzothiazin-1,1-dioxid

Eintopfreaktion A

Zu einer Lösung von
50,1 g (0,5 Mol) 2-Amino-thiazol in
750 ml Essigsäureäthylester werden in der Siedehitze
40 ml (0,5 Mol) Chloracetylchlorid in
60 ml Essigsäureäthylester in 30 min zugetropft und das Reaktionsgemisch 30 min unter Rückfluß nachgerührt. Dabei werden insgesamt
800 ml Essigsäureäthylester abdestilliert. Anschließend werden
240 g (1 Mol) Natrium-Benzoesäuresulfimid × $2H_2O$ und
8,3 g (0,05 Mol) Kaliumjodid, in
434 ml Dimethylformamid gelöst, zugegeben und 45 min bei 90 °C gerührt. Danach wird das Reaktionsgemisch mit
2 l Wasser verdünnt und mit 1 N-Ammoniaklösung neutralisiert. Das ausgefallene Produkt wird isoliert, mit Isopropanol und Petroläther nacheinander gewaschen und getrocknet. Man erhält 116 g Produkt, Schmelzpunkt 247,3 °C.

Eintropfreaktion B

Bei RT werden zu einer gerührten Lösung von
4,48 g (0,04 Mol) Kalium-tert.-butylat in
13,5 ml Dimethylformamid unter $N_2$
3,23 g (0,01 Mol) getrocknetes Reaktionsprodukt der Eintopfreaktion A, in
9,7 ml Dimethylformamid gelöst, zugegeben. Das Reaktionsgemisch wird 30 min bei 55 °C gerührt, anschließend abgekühlt und bei RT mit
3 ml (0,02 Mol) halbkonzentrierter Salzsäure teilweise neutralisiert und 30 min mit
2 ml (0,02 Mol) Dimethylsulfat bei 25 °C umgesetzt. Das Reaktionsgemisch wird schließlich mit
20 ml verdünnter Ammoniaklösung versetzt und in
100 ml verdünnte Salzsäurelösung eingerührt. Man erhält 1,7 g 4-Hydroxy-3-(2-thiazolyl-carbamoyl)-2-methyl-2H-1,2-benzothiazin-1,1-dioxid. Das Produkt wird zur Reinigung aus Aceton umkristallisiert. (Schmelzpunkt 236,8 °C, Z).

**Patentansprüche**

1. Verfahren zur Herstellung von 4-Hydroxy-3-(heterocyclocarbamoyl)-2H-1,2-benzothiazin-1,1-dioxiden der allgemeinen Formel

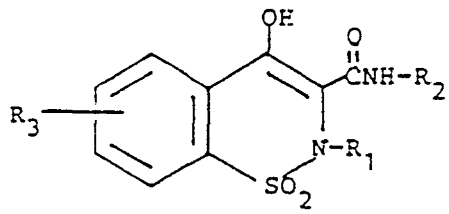

worin bedeuten :

$R_1$ ein Wasserstoffatom oder eine Methylgruppe und

$R_2$ einen gegebenenfalls durch niedere Alkyl- oder Alkoxygruppen substituierten Isoxazol-, Thiazol- oder Pyridinring

$R_3$ ein Wasserstoff- oder Halogenatom oder eine Methylgruppe

oder deren Alkalimetall-, Erdalkalimetall- oder Aminsalzen,

durch Umsetzung einer Verbindung $R_2—NH_2$ mit einem Halogenacetylhalogenid, Umsetzung des erhaltenen Zwischenprodukts mit Na-Benzoesäuresulfimid, Ringerweiterung des Benzoisothiazolrings zum Benzothiazinring und für den Fall, daß $R_2$ ein Isoxazolrest sein soll, Rückumlagerung des bei der Ringerweiterung gebildeten Oxadiazolringes zum Isoxazolring, dadurch gekennzeichnet, daß man (in einer Eintopfreaktion A) die Umsetzung der Verbindung $R_2—NH_2$ mit einem Halogenacetylhalogenid ohne Basenzusatz in siedendem Äthylacetat durchführt, das gebildete Zwischenprodukt ohne Isolierung mit Na-Benzoesäuresulfimid umsetzt und das Reaktionsprodukt isoliert, daß man (in einer Eintopfreaktion B) das Reaktionsprodukt aus der Eintopfreaktion A in einem dipolar aprotonischen Lösungsmittel unter Schutzgas mit einem stark basischen verzweigten Alkalimetall-alkoholat und danach mit soviel Säure, daß in der Reaktionslösung noch etwa 2 Äquivalente Base unneutralisiert bleiben, reagieren läßt, und daß man gegebenenfalls den Benzothiazinring in an sich bekannter Weise N-methyliert.

2. Verfahren nach Anspruch 1, dadurch gekennzeichnet, daß man in der Eintopfreaktion B das Reaktionsprodukt aus der Eintopfreaktion A mit 3-4 Äquivalenten Kalium-tert.-butylat umsetzt.

3. Verfahren nach Anspruch 1, dadurch gekennzeichnet, daß man bei der Eintopfreaktion B 1-2 Äquivalent(e) Säure verwendet.

4. Verfahren nach Anspruch 1 und 3, dadurch gekennzeichnet, daß man bei der Eintopfreaktion B als Säure HCl verwendet.

5. Verfahren nach Anspruch 1 und 2, dadurch gekennzeichnet, daß man die Rückumlagerung bei einer Temperatur von — 5 °C bis + 25 °C durchführt.

**Claims**

1. Process for the preparation of 4-hydroxy-3-(heterocyclocarbamoyl)-2H-1,2-benzothiazine-1,1-dioxides of the general formula :

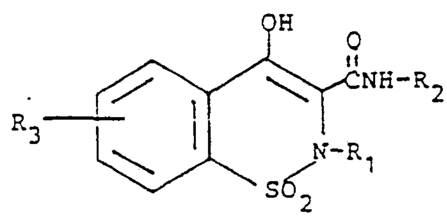

in which

$R_1$ is hydrogen atom or a methyl radical and

$R_2$ is a isoxazolyl-, thiazolyl- or pyridyl radical optionally substituted by lower alkyl- or alkoxy groups and

$R_3$ is a hydrogen or halogen atom or a methyl radical,

and of the alkali metal, alkaline earth metal and amine salts thereof,

by the reaction of a compound $R_2—NH_2$ with a haloacetyl halide, reaction of the intermediate thus obtained with sodium benzoic acid sulphimide, ring expansion of the benzoisothiazole ring to give a benzothiazine ring and in case $R_2$ is to be an isoxazolyl-radical reverse rearrangement of the oxadiazole-ring formed by ring expansion to the isoxazolyl-ring, wherein, in a one-pot reaction A, there is carried out the reaction of a compound $R_2—NH_2$ with a haloacetylhalide without the addition of a base, in boiling ethyl acetate, the intermediate formed is, without isolation, reacted with sodium benzoic acid sulphimide and the reaction product then isolated, whereafter, in a one-pot reaction B, the reaction product from the one-pot reaction A is reacted in a dipolar aprotonic solvent under an atmosphere of a protection gas with a strongly basic alkali-alcoholate and thereafter with so much acid that about 2 equivalents of base remain unneutralised in the reaction solution and, if desired, the benzothiazine ring is N-methylated in the usual manner.

2. Process acording to claim 1, wherein, in one-pot reaction B, the reaction product from one-pot reaction A is reacted with 3 to 4 equivalents of potassium tert.-butylate.

3. Process according to claim 1, wherein in one-pot reaction B, 1 to 2 equivalents of acid are used.

4. Process according to claims 1 and 3, wherein, in one-pot reaction B, hydrochloric acid is used as the acid.

5. Process according to claims 1 and 2 wherein the reverse rearrangement is carried out at a temperature of from — 5 to + 25 °C.

**Revendications**

1. Procédé de préparation de 4-hydroxy-3-(hétérocyclocarbamoyl)-2H-1,2-benzothiazine-1,1-dioxydes de formule générale :

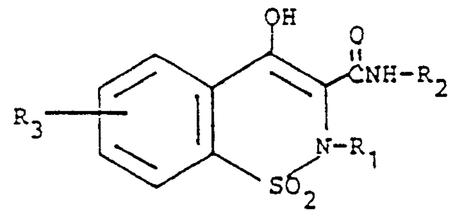

dans laquelle :

$R_1$ représente un atome d'hydrogène ou un groupe méthyle ; et

$R_2$ représente un cycle isoxazolique, thiazolique ou pyridinique éventuellement substitué par des groupes alkyles ou alkoxy inférieurs ;

$R_3$ représente un atome d'hydrogène ou un atome d'halogène ou un groupe méthyle ;

ou de leurs sels de métaux alcalins, de métaux alcalino-terreux ou d'amines, par réaction d'un composé $R_2$—$NH_2$ avec un halogénure d'halogénoacétyle, réaction du produit intermédiaire obtenu avec du benzoate sulfimide de Na, agrandissement du cycle du benzothiazol en cycle de la benzothiazine et, au cas où $R_2$ doit représenter un reste isoxazolique, retransposition du cycle oxadiazolique formé lors par l'agrandissement du cycle en cycle isoxazolique, caractérisé en ce que (dans une réaction unique A) on effectue la réaction du composé $R_2$—$NH_2$ avec un halogénure d'halogénoacétyle, sans addition de base, dans de l'acétate d'éthyle bouillant, en ce que le produit intermédiaire formé, sans séparation, est traité par du benzoate sulfimide de Na et en ce que l'on isole le produit de la réaction, en ce que (dans une réaction unique B) on fait réagir le produit de la réaction unique A dans un solvant aprotonique bipolaire sous atmosphère protectrice avec un alcoolate de métal alcalin ramifié fortement basique et ensuite avec une quantité d'acide suffisante pour que subsistent dans la solution réactionnelle encore 'deux équivalents base non neutralisés et que, éventuellement, d'une façon connue en soi, on procède à la N-méthylation du cycle de la benzothiazine.

2. Procédé selon la revendication 1, caractérisé en ce que dans la réaction unique B, on traite le produit de la réaction unique A par trois à quatre équivalents de tertiobutylate de potassium.

3. Procédé selon la revendication 1, caractérisé en ce que dans la réaction B on utilise un à deux équivalents d'acide.

4. Procédé selon la revendication 1 et 3, caractérisé en ce que dans la réaction unique B on utilise comme acide HCl.

5. Procédé selon les revendications 1 et 2, caractérisé en ce que l'on effectue la retransposition à une température de — 5 °C à + 25 °C.